# EUROPÄISCHE PATENTANMELDUNG

(11) **EP 0 752 251 A2**
(43) Veröffentlichungstag der Anmeldung: **08.01.1997**
(21) Anmeldenummer: 96106985.3
(22) Anmeldetag: 03.05.1996
(51) Int. Cl.: A61M 25/00

(54) **Urologische Dränageeinrichtung mit Ureterkatheter**

(30) Priorität: 06.07.1995 DE 19524591
(71) Anmelder: UROMED KURT DREWS GMBH, D-22113 Oststeinbek (DE)
(72) Erfinder: Drews, Kurt, 22113 Oststeinbek (DE)
(74) Vertreter: Emmel, Thomas, Dipl.-Biol., Dr.

(57) **Zusammenfassung**

Eine urologische Dränageeinrichtung mit einem Ureterkatheter (1) und einem Vorschubschlauch (6), die an einer Kupplungsstelle lösbar verbunden sind durch Aufstecken des im Umfang flexibel erweiterbaren einen an der Kupplungsstelle liegenden Endstückes (8) auf das außen konisch verjüngte andere Endstück (7), und mit einem konzentrisch zum Vorschubschlauch und axial verschiebbar angeordneten Entkupplungsschlauch (10), der in Kupplungsstellung mit seinem distalen Ende gegen das proximale Ende des Katheters stößt, ist dadurch gekennzeichnet, daß das distale Endstück (8) des Vorschubschlauches über das konisch verjüngte proximale Endstück (4) des Kathteters gesteckt ist, wobei der Entkupplungsschlauch innerhalb des Vorschubschlauches (6) angeordnet ist.

## Beschreibung

Die Erfindung betrifft eine urologische Dränageeinrichtung der im Oberbegriff des Anspruches 1 genannten Art.

Derartige Dränageeinrichtungen dienen der Verlegung eines Ureterkatheters, also eines Katheters, der in den Ureter verlegt wird, wobei in Verlegeposition das distale Ende des Katheters in der Niere und das proximale Ende in der Blase liegt. Dabei sind üblicherweise die Endbereiche des Katheters geringelt ausgebildet (Pigtail-Form), um nach der Verlegung den sicheren Sitz zu gewährleisten.

Bei der Verlegung dient der Vorschubschlauch zum Vorschieben des Katheters aus einem in die Blase verlegten endoskopischen Instrument heraus. Da Ureterkatheter in der Regel dann notwendig sind, wenn der Ureter durch einen Stein, eine Verletzung, einen Tumor od. dgl. nicht mehr durchgängig ist, gestaltet sich das Hindurchführen der distalen Katheterspitze durch den Ureter zumeist schwierig, und es sind mehrfache Versuche erforderlich, die auch ein zwischenzeitliches Zurückziehen des Katheters erfordern. Dazu sind Dränageeinrichtungen der eingangs genannten Art derart ausgebildet, daß zum Verlegen der Ureterkatheter mit dem Vorschubschlauch zuglest gekuppelt ist. Dabei muß die Kupplung so ausgebildet sein, daß sie nach beendeter Verlegung leicht gelöst werden kann, um den nicht mehr benötigten Vorschubschlauch herauszuziehen, ohne dabei die gewünschte Endlage des Ureterkatheters zu beeinflussen.

Für die Ausbildung der Kupplungsstelle sind im Stand der Technik eine Reihe von Lösungen bekannt. Eine weit verbreitete Konstruktion gemäß der
DE 38 31 652 A1
sieht eine seitlich lösbare Kupplung vor, die in fluchtender Kupplungslage durch den bei solchen Dränageeinrichtungen stets während des Einführens verwendeten Mandrin gehalten wird.

Eine Dränageeinrichtung der eingangs genannten Art ist aus der
EP 0 365 269 B1, Fig. 5
bekannt. An der Kupplungsstelle ist das flexibel erweiterbare proximale Endstück des Katheters auf das außen konisch verjüngte Endstück des Vorschubschlauches klemmend aufgesteckt. Diese Kupplungsart hat eine Reihe von Vorteilen. Sie ist kostengünstig fertigbar, läßt sich je nach Wunsch mit unterschiedlichen Lösekräften aufstecken und durch den gesondert vorgesehenen Kupplungsschlauch sehr leicht lösen durch Vorschieben des Entkupplungsschlauches gegenüber dem Vorschubschlauch, wodurch das aufgesteckte Endstück des Ureterkatheters abgestoßen wird. Ein weiterer Vorteil ist, daß mit einer solchen Kupplungsstelle die Dränageeinrichtung zum Einspritzen von Kontrastmittel verwendbar ist, was bei der vorgenannten, nicht gattungsgemäßen bekannten Konstruktion nicht möglich ist. Bei der gattungsgemäßen Konstruktion hält die Kupplungsstelle die sichere Verbindung auch bei herausgezogenem Mandrin und ist die Kupplungsstelle flüssigkeitsdicht. Es kann also vom proximalen Ende des Vorschubschlauches her bis in den Ureterkatheter Kontrastmittel gespritzt werden, was für die Verlegung mit Hilfe eines Röntgenapparates von Vorteil ist.

Die bekannte gattungsgemäße Konstruktion hat allerdings auch Nachteile. Bei ihr ist nämlich das aufgeweitete proximale Ende des Katheters über das konische distale Endstück des Vorschubschlauches geschoben, und es ist folglich der Entkupplungsschlauch außen auf dem Vorschubschlauch angeordnet. Dies hat im wesentlichen zwei Nachteile.

Zunächst stört die außen auf dem Vorschubschlauch sitzende aufgeweitete proximale Endfläche des Katheters, die bei Rückwärtsbewegungen der Dränageeinrichtung verhaken kann, beispielsweise am distalen Rand des Endoskopkanales, durch den die Dränageeinrichtung verlegt wird. Tritt hier ein Verhaken beim Rückziehen auf, so kann ungewollt der Vorschubschlauch vom Katheter abgezogen, also die Kupplung gelöst werden. Der noch nicht in Endstellung sitzende Katheter kann dann nur mit einem Zangeninstrument wieder entfernt werden.

Außerdem ist bei dieser Konstruktion die Tatsache ungünstig, daß zum Lösen der Kupplung der außen liegende Entkupplungsschlauch gegenüber dem innen liegenden Vorschubschlauch nach vorn geschoben werden muß. Die dazu erforderliche Handhabung geschieht an den proximalen Enden der beiden Schläuche, wobei das proximale Ende des Vorschubschlauches das proximale Ende des Entkupplungsschlauches überragen muß. Diese Schlauchenden müssen zum Entkuppeln in Axialrichtung auseinander bewegt werden, wozu zwei Hände erforderlich sind.

Die Aufgabe der vorliegenden Erfindung besteht darin, eine Dränageeinrichtung der eingangs genannten Art zu schaffen, die sicherer gegen ungewollte Trennung der Kupplung und einfacher zum Trennen bedienbar ausgebildet ist.

Diese Aufgabe wird erfindungsgemäß mit den Merkmalen des Kennzeichnungsteils des Anspruches 1 gelöst.

Bei dieser Konstruktion ist gegenüber der bekannten gattungsgemäßen Konstruktion die Kupplungsstelle im Grunde nur "umgekehrt" ausgebildet. Es ist also das Endstück des Vorschubschlauches über das konische Endstück des Katheters geschoben und der Entkupplungsschlauch nicht außerhalb, sondern nun innerhalb des Vorschubschlauches angeordnet. Damit werden aber überraschenderweise die beiden oben genannten Nachteile des Standes der Technik sehr wirkungsvoll beseitigt. All der Kupplungsstelle steht nun die distale Endfläche des Vorschubschlauches außen vor und bildet eine Kante nach vorn im Gegensatz zu der nach rückwärts gerichteten Kante nach dem Stand der Technik. Diese nach vorn gerichtete Kante kann nur bei Vorwärtsbewegung der Dränageeinrichtung irgendwo anhaken, was nicht zur Trennung der Kupplung führen kann. Beim Rückziehen der Dränageeinrichtung kann kein Anhaken erfolgen. Ein ungewolltes Entkuppeln während der Verlegearbeit ist also ausgeschlossen. Ferner liegt nun der Entkupplungsschlauch innerhalb des Vorschubschlauches und überragt proximal den Vorschubschlauch. Zum Entkuppeln, wobei wiederum der Entkupplungsschlauch gegenüber dem Vorschubschlauch in distale Richtung zu bewegen ist, werden die beiden Enden dieser Schläuche relativ zueinander bewegt. Dies kann sehr einfach mit einer Hand erfolgen durch Angreifen an entsprechend vorgesehenen Faßstücken an den Enden der Schläuche. Dadurch vereinfacht sich die Bedienung beim Trennen. Es sind nicht mehr zwei Hände erforderlich, sondern es kann mit einer Hand gearbeitet werden, was für den Operateur eine große Erleichterung ist, der nun mit der anderen Hand in gewohnter Weise beispielsweise das Endoskop halten kann, ohne dies zwischenzeitlich an einen Assistenten abgeben zu müssen.

Vorteilhaft sind die Merkmale des Anspruches 2 vorgesehen. Auf diese Weise wird die Umfangsaufweitung des übergesteckten distalen Endstückes des Vorschubschlauches an der Kupplungsstelle durch seine konische Verjüngung ausgeglichen, so daß sich an der Kupplungsstelle keine Durchmessererweiterung ergibt. Es besteht nicht die Gefahr des Klemmens der Kupplungsstelle in einem engen Endoskopkanal.

Vorteilhaft sind dabei die Merkmale des Anspruches 3 vorgesehen. Dadurch, daß die Verjüngung außen sitzt, der Schlauch innen also gerade verläuft, wird das distale Endstück des Vorschubschlauches beim Aufschieben auf das außen konische Endstück des Katheters allmählich elastisch aufgeweitet, so daß durch die Länge des Aufsteckweges die gewünschte Haltekraft eingestellt werden kann.

In der Zeichnung ist die Erfindung beispielsweise und schematisch dargestellt. Es zeigen:
- Fig. 1: eine teilgeschnittene perspektivische Ansicht einer erfindungsgemäßen Dränageeinrichtung und
- Fig. 2: einen vergrößerten Schnitt durch die voneinander gelösten Endstücke des Katheters und des Vorschubschlauches im Kupplungsbereich.

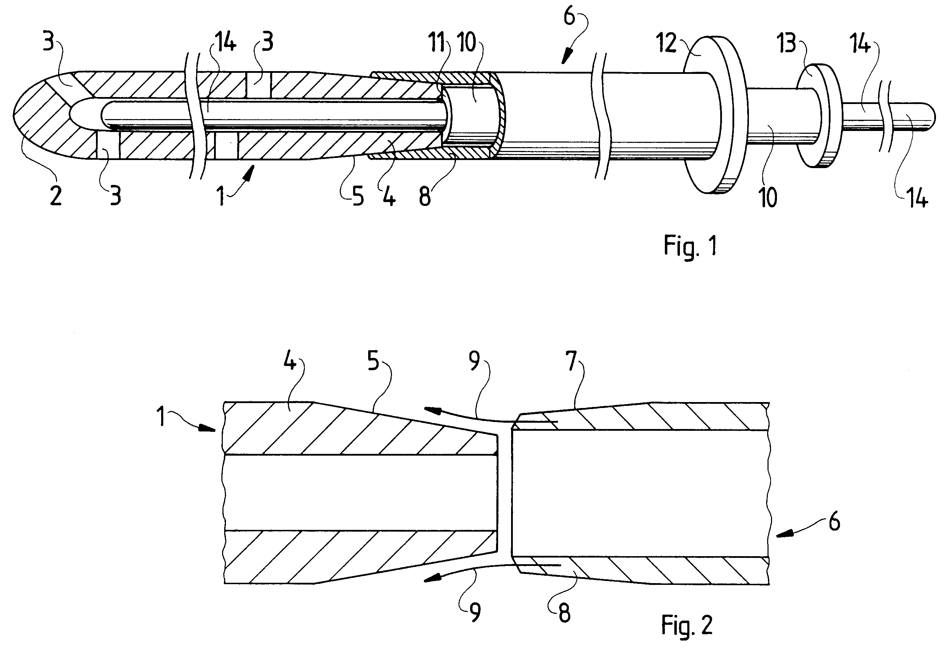

Die in Fig. 1 dargestellte Dränageeinrichtung dient zum Verlegen eines Ureterkatheters 1, der im dargestellten Ausführungsbeispiel an seiner distalen Spitze 2 geschlossen ausgebildet ist, dort aber auch offen sein kann und der wenigstens im distalen und proximalen Endbereich oder auch über die gesamte Länge mit Durchbrechungen 3 gelocht ist. Sein proximales Endstück 4 ist mit einem Außenkonus 5 zum proximalen Ende hin verjüngt ausgebildet.

Ein Vorschubschlauch 6 weist, wie Fig. 2 zeigt, ein ebenfalls außen mit einem Außenkonus 7 verjüngtes distales Endstück 8 auf, das durch Aufschieben in Richtung der Pfeile 9 in distaler Richtung auf den Außenkonus 5 des Ureterkatheters 1 unter elastischer Aufweitung in die in Fig. 1 dargestellte Kupplungsstellung gebracht werden kann, in der das distale Endstück 8 des Vorschubschlauches 6 unter elastischer Spannung festhaltend am Ureterkatheter 1 eine Kupplung bildet.

Im Inneren des Vorschubschlauches 6 ist konzentrisch ein Entkupplungsschlauch 10 angeordnet, wie Fig. 1 zeigt. Der Entkupplungsschlauch 10 stößt mit seiner distalen Endfläche 11 gegen das proximale Ende des Ureterkatheters 1 und überragt das proximale Ende des Vorschubschlauches 6. An den proximalen Enden des Vorschubschlauches 6 und des Entkupplungsschlauches 10 ist jeweils ein im Ausführungsbeispiel scheibenförmig ausgebildetes Griffstück 12 bzw. 13 angeordnet.

In Fig. 1 ist die Dränageeinrichtung in zum Einführen gebrauchsfertig montierter Stellung dargestellt, wobei an der Kupplungsstelle die Ureterschiene 1 mit dem Vorschubschlauch 6 gekuppelt ist. Der Entkupplungsschlauch 10 liegt lose in Anlage am proximalen Ende des Ureterkatheters 1. Es ist ferner ein üblicherweise zum Einführen solcher Katheter verwendeter Mandrin 14 vorgesehen, der durchgehend im Inneren der Dränageeinrichtung verlegt ist, also durch den Entkupplungsschlauch 10 und den Ureterkatheter bis zu dessen Spitze 2 verläuft, und der das proximale Ende des Entkupplungsschlauches 10 nach außen überragt. Der Mandrin dient zur Aussteifung des Ureterkatheters 1 während der Verlegung, um diesen besser führbar zu machen.

Die Verlegung der in Fig. 1 dargestellten Dränageeinrichtung geschieht wie folgt:

In der in Fig. 1 dargestellten Konfiguration wird die Dränageeinrichtung durch den Arbeitskanal eines von außen in die Blase verlegten Endoskopes eingeführt und von dort unter optischer Beobachtung mit der Spitze 2 des Ureterkatheters durch das Ostium, die Eingangsklappe des Ureters, in diesen eingeführt und dann im Ureter bis zur Niere vorgeschoben. Der Operateur hält dabei die dargestellte Dränageeinrichtung an dem außen aus den, Endoskop herausragenden freien Ende des Vorschubschlauches 6 und kann über diesen, der über die Kupplung druck- und zugfest mit dem Ureterkatheter 1 verbunden ist, den Ureterkatheter im Ureter nach Bedarf vor- und zurückschieben, um beispielsweise an Hindernissen oder Engstellen vorbeizukommen.

In jeder beliebigen Zwischenstellung der Verlegung kann der Mandrin 14 herausgezogen werden, ohne daß dabei die Kupplung gelöst wird. Es kann nun vom Ende der Entkupplungsschiene 10 her, die in geeigneter Weise als Spritzenkonus ausgebildet sein kann, Kontrastmittel eingespritzt werden, das aus den Durchbrechungen 3 des Ureterkatheters 1 austritt und mit dem unter Röntgenkontrolle die korrekte Lage des Ureterkatheters überprüft werden kann.

Ist die gewünschte Lage des Ureterkatheters 1 erreicht, dessen distaler Endbereich im Nierenbecken liegt, so wird der Mandrin 14 herausgezogen, und es wird die Kupplung gelöst. Dazu faßt der Operateur die Griffstücke 12 und 13 z.B. in der Weise, daß er mit Zeigefinger und Mittelfinger einer Hand den Vorschubschlauch 6 in Anlage der Finger an dem Griffstück 12 umgreift und mit dem Daumen gegen die proximale Endfläche des Griffstückes 13 drückt. Er kann nun bequem mit den Fingern einer Hand die Griffstücke 12 und 13 gegeneinander drücken. Dadurch drückt bei festgehaltenem Vorschubschlauch 6 der Entkupplungsschlauch 10 mit seiner distalen Endfläche 11 das proximale Endstück 4 des Ureterkatheters 1 aus dem Kupplungseingriff heraus, so daß die Kupplung gelöst wird.

Bei dieser zum Lösen der Kupplung erforderlichen Relativbewegung des Entkupplungsschlauches 10 gegenüber dem Vorschubschlauch 6 ist darauf zu achten, daß nach Möglichkeit die Lage des Ureterkatheters 1 nicht mehr verändert wird. Es wird also bei der Relativbewegung der Griffstücke 12 und 13 gegeneinander dafür Sorge getragen, das Griffstück 13 und somit den Entkupplungsschlauch 10 stillstehend zu halten, während der Vorschubschlauch 6 zurückgezogen wird. Damit wird vermieden, daß die einmal gewählte optimale Lage des Ureterkatheters 1 beim Lösen der Kupplung verändert wird.

Anschließend werden der Vorschubschlauch 6 und der Entkupplungsschlauch 10 beispielsweise zusammen mit dem Endoskop aus der Blase herausgezogen, und die Verlegearbeit ist abgeschlossen.

## Patentansprüche

1. Urologische Dränageeinrichtung mit einem Ureterkatheter (1) und einem Vorschubschlauch (6), die an einer Kupplungsstelle lösbar verbunden sind durch Aufstecken des im Umfang flexibel erweiterbaren einen an der Kupplungsstelle liegenden Endstückes (8) auf das außen konisch verjüngte andere Endstück (4), und mit einem konzentrisch zum Vorschubschlauch (6) und axial verschiebbar angeordneten Entkupplungsschlauch (10), der in Kupplungsstellung mit seinem distalen Ende (11) gegen das proximale Ende (4) des Katheters (1) stößt, **dadurch gekennzeichnet**, daß das distale Endstück (8) des Vorschubschlauches über das konisch verjüngte proximale Endstück (4) des Kathteters (1) gesteckt ist, wobei der Entkupplungsschlauch (10) innerhalb des Vorschubschlauches (6) angeordnet ist.

2. Dränageeinrichtung nach Anspruch 1, **dadurch gekennzeichnet**, daß das distale Endstück (8) des Vorschubschlauches in entkuppelter Stellung konisch verjüngt ausgebildet ist.

3. Dränageeinrichtung nach Anspruch 2, **dadurch gekennzeichnet**, daß die Verjüngung (7) außen angeordnet ist.
